# EUROPÄISCHE PATENTANMELDUNG

(11) **EP 2 030 633 A1**
(43) Veröffentlichungstag der Anmeldung: **04.03.2009**
(21) Anmeldenummer: 08105030.4
(22) Anmeldetag: 13.08.2008
(51) Int. Cl.: A61K 49/22

(54) **Kontrastmittel für die Ultraschalluntersuchung der Prostata und Verfahren zur Diagnose von Prostatakrebs**

(30) Priorität: 03.09.2007 DE 102007041831
(71) Anmelder: Siemens Aktiengesellschaft, 80333 München (DE)
(72) Erfinder: Ergün, Süleyman, 45130, Essen (DE); Singer, Bernhard, 45147, Essen (DE); Tilki, Derya, 81377, München (DE); Fehre, Jens, 91353, Hausen (DE); Nanke, Ralf, 91077, Neunkirchen am Brand (DE)

(57) **Zusammenfassung**

Die Erfindung betrifft ein Kontrastmittel für die Ultraschalluntersuchung der Prostata und ein Verfahren zur Diagnose von Prostatakrebs. Das Kontrastmittel umfasst kontrastverstärkende Partikel (4), an deren Außenseite wenigstens ein Koppelmolekül (8) vorhanden ist, das spezifisch an CEACAM-1 bindet. Bei dem Verfahren wird dem Patienten das Kontrastmittel verabreicht und anschließend eine Sonographie der Prostata durchgeführt.

## Beschreibung

### Kontrastmittel für die Ultraschalluntersuchung der Prostata und Verfahren zur Diagnose von Prostatakrebs

Die Erfindung betrifft ein Kontrastmittel für die Ultraschalluntersuchung sowie ein Verfahren zur Diagnose von Prostatakrebs. Die transrektale Ultraschalluntersuchung (TRUS = transrectal ultrasonography) ist heute neben der digitalrektalen Untersuchung (Tastbefund) und der Bestimmung des prostataspezifischen Antigens (PSA-Bluttest) eine der drei Säulen der gegenwärtigen Prostatakrebsdiagnose im klinischen Alltag. Allerdings weist die Ultraschalluntersuchung wie auch die anderen Standardverfahren eine unzureichende Sensitivität und Spezifität auf. Ursache hierfür ist unter anderem, dass Prostatakarzinome sowohl echoarm als auch echogleich oder echoreicher als gesundes Gewebe sein können. Die Ultraschallsonographie wird bei der Diagnose von Prostatakrebs hauptsächlich bei der Steuerung von Biopsien eingesetzt. Bei einem auffälligen Vorbefund werden unter Ultraschallkontrolle mehrere Stanzzylinder aus der Prostata entnommen, die anschließend histologisch untersucht werden. Da die Karzinome oft nicht im Ultraschallbild gesehen werden können, erfolgt quasi eine blinde Biopsie, d.h. die Proben werden nicht aus verdächtigen Arealen, sondern nach einem vorgegebenen Schema aus allen Bereichen der Prostata entnommen. Dabei wird die Anwendung von Ultraschall nur zur anatomischen Orientierung eingesetzt. Der Erfolg der Biopsie hängt somit von der Trefferwahrscheinlichkeit ab, weshalb viele insbesondere kleine Tumore im Frühstadium unentdeckt bleiben, so dass vielfach Re-Biopsien erforderlich sind.

Für das Wachstum von Tumoren spielt die Neubildung von Blutgefäßen eine wichtige Rolle (Angiogenese / Vaskulogenese), was auch für die Ultraschallbildgebung ausgenutzt werden kann. Mit Hilfe von Doppler-Untersuchungen und dem Einsatz sogenannter Microbubbles lässt sich der verstärkte Blutfluss in den kleinen Blutgefäßen darstellen. Dadurch können gegebenenfalls Tumore oder verdächtige Areale für die Diagnose und die Biopsie besser identifiziert werden als bei der konventionellen B-Bild-Darstellung. Die im Durchschnitt einige µm großen Microbubbles bestehen aus einer Hülle beispielsweise aus Lipiden und sind luft- oder gasgefüllt, was einen starken Kontrast im Ultraschallbild ergibt (direkt oder durch Anregung).

Darüber hinaus laufen derzeit präklinische Studien bezüglich des Einsatzes von sogenannten targeted Microbubbles. Es handelt sich dabei um Microbubbles, auf deren Hülle Koppelmoleküle, beispielsweise Antikörper angebracht sind, die an bestimmte Zielmoleküle im Körper andocken sollen. Ziel ist es, dass sich die Microbubbles in ganz bestimmten Zielgebieten anreichern und dadurch eine ultraschallgestützte Bildgebung dieses Areals ermöglichen. Als mögliche Zielmoleküle werden prostata-unspezifische, die Angiogenese anzeigende Zielmoleküle (wie z.B. VEGF-R2(KDR) oder alpha(v)-beta(3)-Integrin) untersucht.

Aufgabe der Erfindung ist es, ein Kontrastmittel für die Ultraschalluntersuchung der Prostata und ein Verfahren zur Diagnose von Prostatakrebs bereitzustellen, mit denen ein Prostatatumor bereits im frühen Stadium erkannt bzw. behandelt werden kann.

Diese Aufgabe wird durch ein Kontrastmittel nach Anspruch 1 bzw. durch ein Verfahren nach Anspruch 9 gelöst. Das erfindungsgemäße Kontrastmittel enthält kontrastverstärkende Partikel, an deren Oberfläche wenigstens ein Koppelmolekül vorhanden ist, das spezifisch an CEACAM-1 bindet. Die Erfindung stützt sich dabei auf Forschungsergebnisse von Tilki et al. (Oncogene (2006)25,4965-4974), gemäß denen CEACAM-1 bei einer gesunden Prostata nur in deren Epithel, nicht jedoch in den Blutgefäßen der Prostata vorkommt. In einer frühen Phase der Tumorentwicklung, etwa im Stadium der hgPIN (high grade epithelial prostatic neoplasia), in welcher der Tumor noch keine eigenen Gefäße ausbildet, ist die Expression von CEACAM-1 im Prostataepithel herunterreguliert, während sie im Endothel angrenzender kleiner Blutgefäße des gesunden Gewebes hochreguliert ist. Dieser Effekt wird nun erfindungsgemäß zur Erkennung eines Krebsfrühstadiums ausgenutzt. Im Gegensatz zu einer gesunden Prostata ergibt sich bei Vorhandensein eines Tumors im Frühstadium ein völlig anderes Anreicherungsbild. Hier erfolgt eine Konzentration des Kontrastmittels im Bereich des Tumors, so dass dieser im Ultraschallbild sichtbar ist.

Für ein erfindungsgemäßes Verfahren zur Diagnose von Prostatakrebs, bei dem dem Patienten ein Kontrastmittel nach einem der vorhergehenden Ansprüche verabreicht und anschließend eine Sonographie der Prostata durchgeführt wird, gelten die obigen Ausführungen analog.

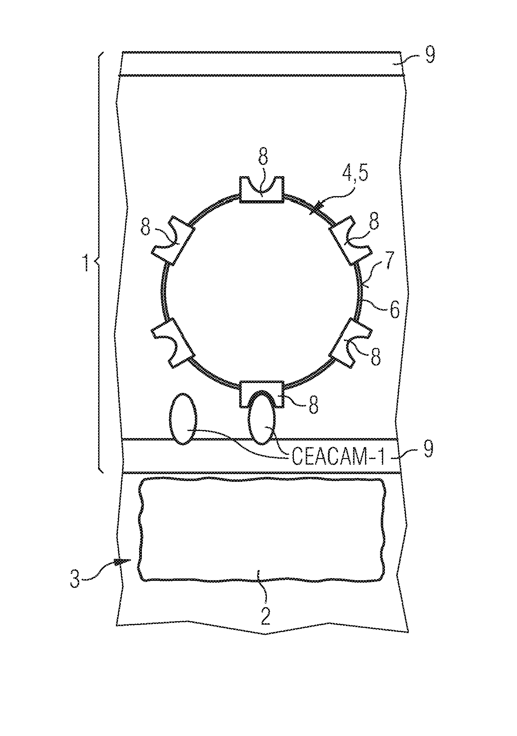

In den Unteransprüchen sind vorteilhafte Weiterbildungen angegeben, die bei der folgenden, auf die beigefügte Abbildung Bezug nehmenden Beschreibung der Erfindung berücksichtigt werden.

Die Abbildung ist eine schematisierte Darstellung eines ein Blutgefäß 1 und einen Tumor 2 aufweisenden Bereichs der Prostata 3, welche den Aufbau des Kontrastmittels und dessen Anreicherung in den Blutgefäßen 1 verdeutlicht. Ein über die Blutbahn etwa nach z.B. intravenöser Verabreichung der Prostata 3 zuführbares Kontrastmittel enthält Partikel 4, die bei der Sonographie ein Ultraschallecho ergeben, das deutlicher ausfällt, als das Echo eines Gewebebereichs der Prostata 3 bzw. des Tumors 2. Besonders bevorzugt sind Microbubbles 5 als kontrastverstärkende Partikel. Es handelt sich dabei um einige Mikrometer große, mit Luft oder einem anderen Gas gefüllte Bläschen mit einer Hülle 6 beispielsweise aus Lipiden. Neben Microbubbles können aber auch aus echoreichen Substanzen, beispielsweise von Biopolymeren gebildete Partikel verwendet werden. Als geeignete Materialien sind insbesondere Alginat und Chitin zu nennen.

An die Außenseite 7 der Hülle sind Koppelmoleküle 8 mittel- oder unmittelbar gebunden, die spezifisch an CEACAM-1-Moleküle (carzinoembryonic antigen-related cell adhesion molecules) binden. Diese Moleküle bilden sich in Blutgefäßen 1 aus, die dem Tumorgewebe benachbart sind. Es kommt dabei zu einer Anreicherung von Kontrastmittel-Partikeln in dem dem Tumor 2 benachbarten Gewebe der Prostata 3. Die CEACAM-1-Moleküle sind in der Wand 9, und zwar in deren Endothel angeordnet, und sind daher über die Blutbahn erreichbar. Als Koppelmoleküle 8 dienen vor allem CEACAM-1-Antikörper. Es sind aber auch gentechnisch hergestellte lösliche Formen von CEACAM-1 geeignet. Ebenfalls geeignet sind Aptamere, Spiegelmere und/oder Anticaline. Aptamere sind kurze, stabile und spezifisch bindende RNA-Ketten, Spiegelmere sind deren spiegelbildliche Pendants. Bei den Anticalinen handelt es sich um einzelne Polypeptidketten mit ca. 180 Aminosäuren, die ähnlich spezifische Bindungseigenschaften aufweisen wie Antikörper, die aber leichter herstellbar sind diese.

Eine Ultraschalluntersuchung kann beispielsweise wie folgt ablaufen: Dem Patienten wird das Kontrastmittel injiziert. Die Prostata wird mit Hilfe der Ultraschallsonographie überwacht, wobei zweckmäßigerweise eine Rektalsonde verwendet wird, die in bekannter Weise eine Ultraschallsende- und - Empfangseinheit umfasst. Nach Verstreichen einer gewissen Zeit hat sich das Kontrastmittel im Körper verteilt, wobei es aufgrund der Bindung zwischen einem Koppelmolekül 8 und CEACAM-1 zu einer Anreicherung in Gewebebereichen kommt, in deren Gefäßbett CEACAM-1 vorkommt. Bei Vorhandensein eines Tumors im Frühstadium ist eine Kontrastmittel-Anreicherung im Bereich des Tumors, d.h. in diesem benachbarten kleinen Gefäßen zu beobachten. Die Kontrastmittelpartikel 4 sind echoreicher als das sie umgebenden Gewebe. Es werden dabei noch relativ kleine, mit herkömmlichen Methoden nicht erkennbare Tumorherde sichtbar.

## Patentansprüche

1. Kontrastmittel für die Ultraschalluntersuchung der Prostata, umfassend kontrastverstärkende Partikel (4), an deren Außenseite (7) wenigstens ein Koppelmolekül (8) vorhanden ist, das spezifisch an CEACAM-1 bindet.

2. Kontrastmittel nach Anspruch 1, bei dem die kontrastverstärkenden Partikel (4) von Microbubbles (5) gebildet sind.

3. Kontrastmittel nach Anspruch 1, bei dem die kontrastverstärkenden Partikel (4) von Biopolymeren gebildet sind.

4. Kontrastmittel nach Anspruch 3, bei dem die Partikel (4) zumindest teilweise aus Chitin bestehen.

5. Kontrastmittel nach Anspruch 3, bei dem die Partikel zumindest teilweise aus Alginat bestehen.

6. Kontrastmittel nach einem der vorhergehenden Ansprüche, bei dem als Koppelmoleküle (8) CEACAM-1-Antikörper vorhanden sind.

7. Kontrastmittel nach einem der vorhergehenden Ansprüche, bei dem als Koppelmoleküle (8) gentechnisch hergestellte lösliche Formen von CEACAM-1 vorhanden sind.

8. Kontrastmittel nach einem der vorhergehenden Ansprüche, bei dem als Koppelmoleküle (8) Aptamere, Spiegelmere und/oder Anticaline vorhanden sind.

9. Verfahren zur Diagnose von Prostatakrebs, bei dem dem Patienten ein nach einem der vorhergehenden Ansprüche verabreicht und anschließend eine Sonographie der Prostata durchgeführt wird.

10. Verfahren nach Anspruch 9, bei dem eine rektale Sonographie durchgeführt wird.
